**Europäisches Patentamt**

**European Patent Office**

**Office européen des brevets**

(19)

(11) Veröffentlichungsnummer: **0 119 984**
**A1**

(12) ## EUROPÄISCHE PATENTANMELDUNG

(21) Anmeldenummer: **84890031.2**

(22) Anmeldetag: **21.02.84**

(51) Int. Cl.³: **C 12 M 1/22**

(30) Priorität: **22.02.83 AT 600/83**

(43) Veröffentlichungstag der Anmeldung: **26.09.84**
**Patentblatt 84/39**

(84) Benannte Vertragsstaaten: **BE CH DE FR GB IT LI LU NL SE**

(71) Anmelder: **C.A. GREINER & SÖHNE GESELLSCHAFT M.B.H., Greinerstrasse 70, A-4550 Kremsmünster Oberösterreich (AT)**

(72) Erfinder: **Greiner, Peter, Dr., Greinerstrasse 70, A-4550 Kremsmünster Oberösterreich (AT)**
Erfinder: **Konrad, Franz, Preising 106, A-4844 Regau Oberösterreich (AT)**

(74) Vertreter: **Krause, Ernst, Dipl.-Ing. et al, Dipl.- Ing. Krause Ernst Dipl. Ing. Casati Wilhelm Patentanwälte Amerlingstrasse 8, A-1061 Wien (AT)**

(54) Petrischale.

(57) Der Unterteil (2) dieser Petrischale (1), die einen Nährboden aufweist, ist mit einem Deckel (3) verschliessbar, wobei Unterteil (2) und Deckel (3) aus Kunststoff bestehen und aus einer bevorzugt transparenten Folie tiefgezogen sind. Zwischen Unterteil (2) und Deckel (3) ist eine heisssiegelung vorgesehen, wobei insbesondere der Deckel (3) eine Heisssiegelschicht an seiner gesamten Innenfläche aufweist. Der Unterteil (2) weist am Umfang seiner Öffnung einen Siegelrand (6) auf, der in einer parallel zum boden (7) liegenden Ebene verläuft, und am Rand des Unterteiles (2) oder bzw. und des Deckels (1) sind Positionierglieder (12, 19) ausgebildet, mit deren Hilfe, nach dem Lösen der Versiegelung, der Deckel (3) sowohl wieder anliegend an den Unterteil (2) als auch im Abstand von demselben, unter Belassung von Lüftungsöffnungen, aufsetzbar ist.

Zur einfachen Herstellung sowie Handhabung dieser Petrischale sowie zur Materialeinsparung sind die Positionierglieder (12, 19) im Bereich des Siegelrandes (6) des Unterteiles (2) und des Siegelflansches (9) des Deckels (3) angeordnet.

0119984

## Petrischale

Gegenstand eines älteren Vorschlages der Anmeldung ist eine einen Nährboden aufweisende Petrischale, deren Unterteil mit einem Deckel verschließbar ist, wobei Unterteil und Deckel aus Kunststoff bestehen und aus einer bevorzugt transparenten Folie tiefgezogen sind; diese Petrischale ist dadurch gekennzeichnet, daß zwischen Unterteil und Deckel eine an sich bekannte Heißsiegelung vorgesehen ist.

Weitere Merkmale nach diesem Vorschlag bestehen darin, daß der Deckel eine Heißsiegelschicht an seiner gesamten Innenfläche aufweist, und darin, daß der Unterteil am Umfang seiner Öffnung einen Siegelrand aufweist, der vorzugsweise in einer parallel zum Boden liegenden Ebene verläuft. Ferner sieht dabei eine besonders vorteilhafte Ausführungsform vor, daß am Rand des Unterteils oder bzw. und des Deckels außerhalb der Zone der Heißsiegelung Positionierglieder ausgebildet sind, durch die, nach dem Lösen der Heißsiegelung, der Deckel sowohl wieder anliegend an den Unterteil als auch im Abstand von demselben, unter Belassung von Lüftungsöffnungen, aufsetzbar ist.

Bei der bisherigen Ausführung solcher Petrischalen befanden sich die Positionierglieder außerhalb der Zonen der Heißsiegelung; der Deckel mußte sohin in einem zylindrischen oder kegelmantelförmigen Wandteil enden, an welchen die Positionierglieder ausgebildet wurden, was für die Herstellung komplizierte und Hinterschneidungen aufweisende Formen erforderte und erhöhten Materialbedarf nötig machte.

Die Erfindung stellt sich die Aufgabe, die Petrischale der bisher vorgeschlagenen Art in ihrer Herstellung und Handhabung, unter Vermeidung unnötigen Materialaufwandes, zu vereinfachen.

Demzufolge besteht die Lösung dieser Aufgabe bei einer solchen Petrischale darin, daß die Positionierglieder im Bereich des Siegelrandes des Unterteiles und des Siegelflansches des Deckels angeordnet sind. In dieser Weise ergibt sich eine besonders einfache und materialsparende Ausgestaltung der beiden Teile der Petrischale, Unterteil und Deckel, da der Siegelrand an diesen beiden Teilen, an den entsprechenden horizontalen Ringflächen derselben, nun zusätzlich die Positionierglieder aufweist.

Die Positionierglieder bestehen nach einem weiteren Merkmal der Erfindung aus ineinander passenden Vorragungen und Vertiefungen. Besonders vorteilhafterweise ist vorgesehen, daß die Positionierglieder die Form von länglichen, bevorzugt in Umfangsrichtung verlaufenden Rippen aufweisen.

Weitere Einzelheiten und Vorteile der Erfindung ergeben sich aus der folgenden Beschreibung einiger Ausführungsbeispiele anhand der Zeichnung; es zeigen:

Fig. 1 einen Axialschnitt durch die Schale mit heißgesiegeltem Deckel,

Fig. 2 eine ähnliche Darstellung bei verdreht wiederaufgesetztem Deckel unter Belassung von Zirkulationsöffnungen,

Fig. 3, Teilaufsichten des Deckels unter Zuordnung zu den Fig. 1 bzw. 2,

Fig. 4 einen Schnitt nach der Linie IV-IV der Fig. 3 und die

Fig. 5 - 7 zu Fig. 3 ähnliche Darstellungen von drei Abänderungen der Positionierglieder.

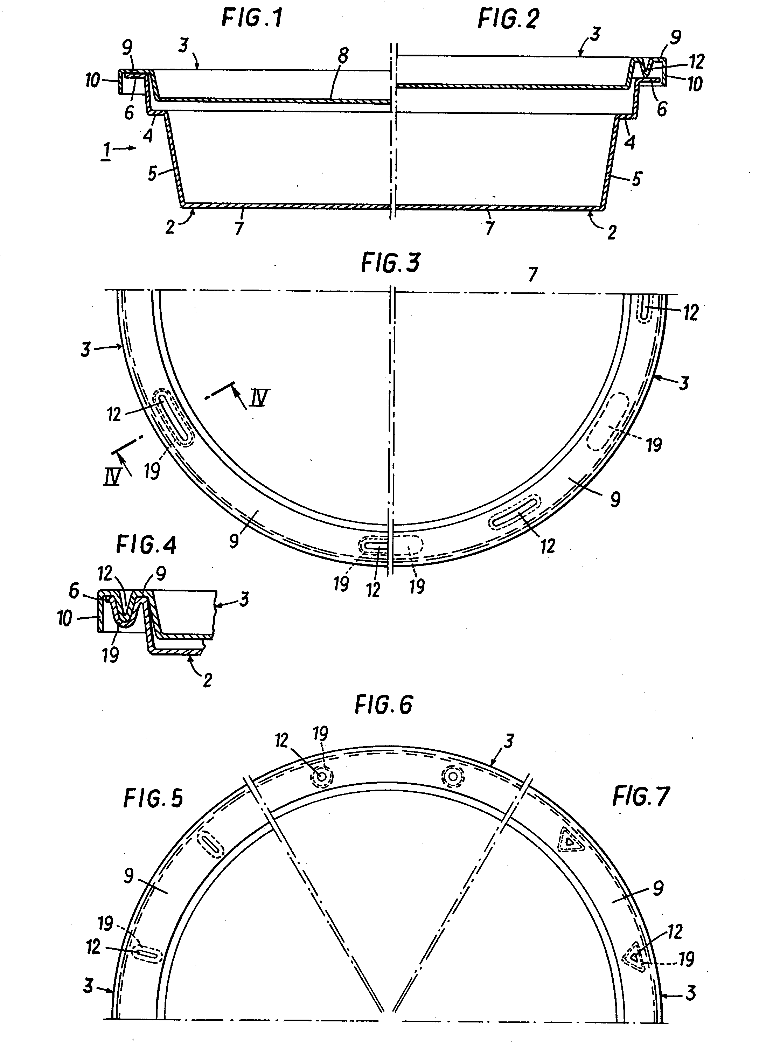

Die aus zwei tiefgezogenen, insbesondere transparenten Kunststoffteilen bestehende Petrischale 1 weist bei dem Beispiel nach den Fig. 1 - 3 einen Unterteil 2 und einen Deckel 3 auf. Der Unterteil 2 besitzt, anschließend an eine Schulter 4 seiner sich konisch nach oben erweiternden Wandung 5, an seiner Öffnung einen Siegelrand 6, der sich parallel zum Boden 7 erstreckt. Der Deckel 3, der an seiner Innenseite mit einer (nicht dargestellten) Schicht aus heißsiegelfähigem Werkstoff versehen ist, besitzt, anschließend an seinen Mittelteil 8, einen entsprechend den Teilen 4 und 6 geformten Siegelflansch 9, der sich außen in einen zylindrischen Mantel 10 fortsetzt. Der Siegelflansch 9 weist dabei, wie aus Fig. 3 hervorgeht, über den Umfang verteilte, als Positionierglieder dienende Vorragungen in Form von sich in Umfangsrichtung erstreckenden Längsrippen auf, die nach unten vorstehen, und diesen Vorragungen 12 sind entsprechende längliche Vertiefungen 19 im Siegelrand 6 des Unterteiles 2 zugeordnet, in die sie eingreifen (Fig. 4).

Die Anordnung ist dabei so getroffen, daß beim erstmaligen Verschließen der Schale 1, also beim Aufsetzen des Deckels 3 auf den mit dem (nicht dargestellten) Nährboden versehenen Unterteil 2, die Vorragungen 12 in die Vertiefungen 19 eintreten, sodaß in der in den Fig. 1 und 3 links bzw. in Fig. 4 ersichtlichen Stellungen der Teile 2, 3 die Heißsiegelung erfolgt. In diesem Zustand ist die Schale sehr lange Zeit lagerbar, unter sterilem Abschluß ihres Inneren gegenüber der Umgebung.

Nach dem ersten Abnehmen des Deckels 3 vom Unterteil 2, unter Zerstörung der Heißsiegelung, und nach der Beimpfung läßt sich der Deckel 3 wieder auf den Unterteil 2 aufsetzen, und zwar in zweierlei Weise: entweder einerseits in gegenüber der ursprünglichen Lage verdrehter Stellung, so daß durch einfaches Auflegen die Vorragungen 12, wie in Fig. 2 ersichtlich, auf dem Siegelrand 6 aufruhen, wodurch zwischen benachbarten Vorragungen 12 Luftschlitze freibleiben, durch die hindurch eine Luftzirkulation von aeroben Kleinlebewesen erfolgen kann, oder aber anderseits in der ursprünglichen Lage genau entsprechender Stellung, so daß durch gänzliches Niederdrücken des Deckels 3 auf den Unterteil 2, wie beim erstmaligen Verschließen der Schale, die Vorragungen 12 in die zugehörigen Vertiefungen 19 eintreten und dann der Siegelrand 6 wieder am Siegelflansch 9 dichtend anliegt, im Falle der Züchtung von anaeroben Kleinlebenwesen. Nach Belieben kann später der Deckel 3 neuerlich abgehoben und in der einen oder anderen Weise wiederaufgesetzt werden.

Im Rahmen der Erfindung sind mannigfaltige Abänderungen an den in Fig. 1 - 3 dargestellten und eben beschriebenen Positioniergliedern 12, 19 möglich; einige solcher Beispiele sind in den Fig. 5 - 7 dargestellt.

Bei der Ausführung nach Fig. 5 weisen die Teile 12, 19 die Form von Querrippen auf, die sich über die ganze Breite des Siegelrandes 6 erstrecken; bei der Ausführung nach Fig. 6 sind sie halbkugelig ausgebildet, und bei jener nach Fig. 7 in Form dreiseitiger Prismen. Beliebig andere Gestaltungen

0119984

dieser Vorragungen und Vertiefungen als Positionierglieder sind denkbar.

Weitere Abänderungen an den beschriebenen und dargestellten Ausführungsbeispielen sind möglich. So kann etwa auch der Deckel nicht flächenhaft mit dem heißsiegelfähigen Werkstoff beschichtet sein, sondern nur im Bereich des Siegelflansches. Anstelle am Deckel kann eine begrenzte Zone dieses Werkstoffes auch auf dem Siegelrand des Unterteiles liegen. Das Versiegeln des Deckels kann mit Hilfe eines Klebers durch Heißsiegeln oder aber durch Verschweißen erfolgen.

0119984

Patentansprüche:

1. Einen Nährboden aufweisende Petrischale, deren Unterteil mit einem Deckel (3) verschließbar ist, wobei Unterteil (2) und Deckel (3) aus Kunststoff bestehen und aus einer bevorzugt transparenten Folie tiefgezogen sind. Zwischen Unterteil (2) und Deckel(3) ist eine Heißsiegelung vorgesehen, wobei insbesondere der Deckel (3) eine Heißsiegelschicht an seiner gesamten Innenfläche aufweist. Der Unterteil (2) weist am Umfang seiner Öffnung einen Siegelrand (6) auf, der in einer parallel zum Boden (7) liegenden Ebene verläuft, und am Rand des Unterteiles (2) oder bzw. und des Deckels (1) sind Positionierglieder (12, 19) ausgebildet,mit deren Hilfe, nach dem Lösen der Versiegelung, der Deckel (3) sowohl wieder anliegend an den Unterteil (2) als auch im Abstand von demselben, unter Belassung von Lüftungsöffnungen, aufsetzbar ist, dadurch gekennzeichnet, daß die Positionierglieder (12, 19) im Bereich des Siegelrandes (6) des Unterteiles (2) und des Siegelflansches (9) des Deckels (3) angeordnet sind.

2. Petrischale nach Anspruch 1, dadurch gekennzeichnet, daß die Positionierglieder (12, 19) aus ineinander passenden Vorragungen (12) und Vertiefungen (19) bestehen.

3. Petrischale nach Anspruch 2, dadurch gekennzeichnet, daß die Positionierglieder die Form von länglichen, bevorzugt in Umfangsrichtung verlaufenden Rippen (12, 19) aufweisen (Fig. 3).

0119984

FIG.1    FIG.2

FIG.3

FIG.4

FIG.6

FIG.5    FIG.7

Europäisches
Patentamt

**EUROPÄISCHER RECHERCHENBERICHT**

0119984

Nummer der Anmeldung

EP 84 89 0031

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | US-A-2 701 229 (G.N. SCHERR) <br> * Figuren; Ansprüche * | 1,2 | C 12 M 1/22 |
| A | | 3 | |
| | --- | | |
| Y | FR-A-1 572 527 (P. MALAGUTI) <br> * Ansprüche; Figuren * | 1,2 | |
| | --- | | |
| A | FR-A-2 436 084 (MANUDO SA) <br> * Figuren; Ansprüche 1,3,5,6 * | 1 | |
| | ----- | | |
| | | | **RECHERCHIERTE SACHGEBIETE (Int. Cl. ³)** |
| | | | C 12 M |

Der vorliegende Recherchenbericht wurde für alle Patentansprüche erstellt.

| Recherchenort <br> DEN HAAG | Abschlußdatum der Recherche <br> 23-05-1984 | Prüfer <br> COUCKE A.O.M. |
|---|---|---|